# EUROPÄISCHE PATENTSCHRIFT

(11) **EP 2 389 881 B1**
(45) Veröffentlichungstag und Bekanntmachung des Hinweises auf die Patenterteilung: **11.12.2013**
(21) Anmeldenummer: 10163966.4
(22) Anmeldetag: 26.05.2010
(51) Int. Cl.: A61B 17/3203, A61M 1/00, A61B 17/32

(54) **Vorrichtung zum flüssigkeitsstrahlunterstützten Trennen und Absaugen von Gewebezellen aus einer biologischen Struktur**
Device for separating and draining tissue cells from a biological structure using a liquid jet
Dispositif de séparation par jet de liquide et d'aspiration de cellules tissulaires à partir d'une structure biologique

(43) Veröffentlichungstag der Anmeldung: 30.11.2011
(73) Patentinhaber: Human Med AG, 19061 Schwerin (DE)
(72) Erfinder: Kensy, Arnd, 14552 Michendorf - OT Wilhelmshorst (DE); Winkler, Konrad-Wenzel, 19417 Warin (DE)
(74) Vertreter: Gulde Hengelhaupt Ziebig & Schneider

(56) Entgegenhaltungen:
- EP-A1- 1 810 623
- DE-A1-102008 027 486
- US-A- 4 662 367
- US-A- 5 078 603
- US-A- 5 441 410
- US-A- 5 724 988
- US-A1- 2008 234 699
- US-A1- 2008 243 028

## Beschreibung

Die Erfindung betrifft eine Vorrichtung zum flüssigkeitsstrahlunterstützten Trennen und Absaugen von Gewebezellen aus einer biologischen Struktur, mit einem Druckerzeuger zum Versorgen eines Applikators der Vorrichtung mit einem definierten Flüssigkeitsstrahl, mit einer Saugeinrichtung zum Entfernen der abgetrennten Gewebezellen und der verwendeten Flüssigkeit aus der biologischen Struktur, wobei die Saugeinrichtung eine Unterdruckquelle und eine Saugleitung zum Verbinden des Applikators mit der Unterdruckquelle umfasst, wobei in die Saugleitung ein Gewebezellensammler eingebunden ist.

Eine Vorrichtung der gattungsgemäßen Art ist beispielsweise aus WO 2009/149691 A2 bekannt. Hier ist eine Vorrichtung zum Trennen von Gewebezellen aus einer Flüssigkeit, bestehend aus einem unter Vakuum stehenden Gewebezellensammler mit einer Filtereinheit, die den Sammelbehälter in einem unteren Sammelraum für die Flüssigkeit, einen mittleren Sammelraum für die Gewebezellen und einen oberen Vakuumraum aufteilt, bekannt, wobei der untere Sammelraum für die Flüssigkeit und der obere Vakuumraum am Sammelraum für die Gewebezellen vorbei miteinander verbunden sind.

Mittels dieser bekannten Vorrichtung ist es möglich, Gewebezellen, insbesondere Fettgewebe, aus einem menschlichen Körper abzusaugen und dieses Fettgewebe in dem Sammelbehälter zu sammeln. Hierbei erfolgt eine Trennung des Fettgewebes von der Arbeitsflüssigkeit. Dieses Fettgewebe steht somit für eine weitere Be- und/oder Verarbeitung, beispielsweise zur Wiederimplantation in den menschlichen Körper an anderer Stelle zur Verfügung.

Aus US 2008/243028 A1 ist ein Gewebezellensammler bekannt, der mehrere Filterebenen umfasst. Hierbei sind mehrere Filter beabstandet zueinander angeordnet, die jeweils eine unterschiedliche Porengröße besitzen. Die Filter sind alle in den Gewebezellensammler integriert.

Für verschiedene Anwendungsmöglichkeiten ist es wünschenswert, dass das Fettgewebe in möglichst reiner Form, das heißt ohne unerwünschte Gewebeanteile, beispielsweise durch mit abgetrennte und abgesaugte Bindegewebeanteile, zur Verfügung steht.

Der Erfindung liegt daher die Aufgabe zugrunde, eine Vorrichtung der gattungsgemäßen Art zu schaffen, die sich durch einen einfachen Aufbau auszeichnet und mittels der Gewebezellen, insbesondere Fettzellen, aus einer biologischen Struktur getrennt und abgesaugt werden können, wobei unerwünschte Gewebeanteile in den gesammelten Gewebezellen weitgehend vermieden werden.

Erfindungsgemäß wird diese Aufgabe mittels einer Vorrichtung mit den in Anspruch 1 genannten Merkmalen gelöst. Dadurch, dass dem Gewebezellensammler eine Fangeinrichtung zum Auffangen von Bindegewebe oder dergleichen aus dem Flüssigkeits-Gewebezellen-Gemisch vorgeschaltet und eine Filtereinrichtung (Sieb) nachgeschaltet ist, wobei die Fangeinrichtung vorzugsweise in den Strömungsweg des Flüssigkeits-Gewebezellen-Gemisches einkragende Fangstrukturen aufweist, ist vorteilhaft möglich, in besonders einfacher Weise eine Trennung der entnommenen Gewebezellen von der Flüssigkeit zu erreichen. Das Vorschalten der Fangeinrichtung ist wichtig, um die dem Sammelraum nachgeschaltete Filtereinrichtung (Sieb) nicht zu verstopfen. Die entnommenen Gewebezellen sind von unerwünschten Bestandteilen, beispielsweise Bindegewebe oder dergleichen, zusätzlich gereinigt. Hierdurch ist die Bereitstellung von beispielsweise einem menschlichen Körper entnommenen Gewebezellen, insbesondere Fettgewebezellen, mit einem hohen Reinheitsgrad möglich.

In bevorzugter Ausgestaltung der Erfindung ist vorgesehen, dass die Fangstrukturen von Bartenstrukturen gebildet sind. Hierdurch lässt sich in besonders einfacher und effektiver Weise ein Herausfiltern von Bindegewebe oder dergleichen aus dem Flüssigkeits-Gewebezellen-Gemisch erreichen.

In weiterer bevorzugter Ausgestaltung der Erfindung ist vorgesehen, dass die Bartenstrukturen in einer Saugleitung, insbesondere zwischen einem Applikator der Vorrichtung und dem Gewebezellensammler, angeordnet sind. Hierdurch wird vorteilhaft möglich, den Aufbau der Vorrichtung insgesamt sehr kompakt zu gestalten und die Anordnung eines zusätzlichen Bauteiles zu vermeiden. Da die Saugleitung üblicherweise Bestandteil eines nur einmal zu verwendenden Sets der gesamten Vorrichtung ist, kann die Filtereinrichtung in der Saugleitung auch nur zum einmaligen Gebrauch ausgelegt sein. Hierdurch kann der Aufbau relativ einfach und zweckoptimiert erfolgen. Eine Reinigung ist nicht erforderlich, da die abgefilterten Bindegewebe oder dergleichen gemeinsam mit dem Einwegset entsorgt werden können.

Ferner ist in bevorzugter Ausgestaltung der Erfindung vorgesehen, dass die Bartenstrukturen von plattenförmigen Körpern gebildet sind, deren Ebenen in Strömungsrichtung des Flüssigkeits-Gewebezellen-Gemisches orientiert sind. Hierdurch ist vorteilhaft möglich, dass die plattenförmigen Körper einen möglichst geringen Strömungswiderstand erzeugen und gleichzeitig die zu sammelnden Gewebezellen passieren lassen. Die in ihrer Struktur größeren Bindegewebe oder dergleichen bleiben an den plattenförmigen Körpern hängen und werden somit ausgefiltert.

Ferner ist in bevorzugter Ausgestaltung der Erfindung vorgesehen, dass die plattenförmigen Körper sich radial nach innen verjüngen und insbesondere sich bis zu einer gedachten Mittellinie der Saugleitung erstrecken. Hierdurch wird vorteilhaft erreicht, dass einerseits ein genügend großer Strömungsquerschnitt zum Durchtritt des Flüssigkeits-Gewebezellen-Gemisches erhalten bleibt und die plattenförmigen Körper radial nach innen ragende hakenförmige Gestalt aufweisen, die ein besonders gutes Herausfiltern von Bindegewebe oder dergleichen aus dem Flüssigkeits-Gewebezellen-Gemisch ermöglichen.

In weiterer bevorzugter Ausgestaltung der Erfindung ist vorgesehen, dass in Strömungsrichtung hintereinander wenigstens zwei Gruppen von plattenförmigen Körpern angeordnet sind, wobei insbesondere die Gruppen der plattenförmigen Körper koaxial zueinander versetzt angeordnet sind. Hierdurch wird eine besonders effektive Filtereinrichtung geschaffen, die einerseits eine geringe Beeinflussung des Strömungsquerschnittes der Saugleitung gewährleistet und andererseits durch versetzt zueinander angeordnete Gruppen der plattenförmigen Körper den gesamten Querschnitt der Saugleitung abdeckt, so dass eine besonders wirkungsvolle Abfilterung der Bindegewebe oder dergleichen möglich ist.

Darüber hinaus ist in bevorzugter Ausgestaltung der Erfindung vorgesehen, dass die Fangstrukturen von der Hauptachse der Fangeinrichtung in Strahlen vom Zentrum nach außen zur Wandung gerichtet sind, wobei diese vorzugsweise in aufeinander folgenden Reihen angeordnet und weiter bevorzugt spiralförmig gedreht sind. Hierdurch lässt sich eine sehr effektive Fangstruktur aufbauen, mittels derer Bindegewebe oder dergleichen sehr effektiv aus dem Flüssigkeits-Gewebezellen-Gemisch herausgefiltert werden kann, ohne zu verstopfen. Ferner ist bevorzugt, wenn die Fangstrukturen von einer Vielzahl bürstenartig angeordneter langgestreckter, insbesondere strangförmiger Gütern gebildet sind. Hierdurch lässt sich sehr vorteilhaft eine der gegebenen Strömungsgeschwindigkeit des Flüssigkeits-Gewebezellen-Gemisches angepasste Herausfilterung der Gewebezellen oder dergleichen erreichen.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen näher erläutert. Es zeigen:
- Figur 1: eine schematische Darstellung einer Einrichtung zum Flüssigkeitsstrahltrennen mit integrierter Vorrichtung zum Trennen von Gewebezellen aus einer Flüssigkeit;
- Figur 2: eine schematische Schnittdarstellung durch einen Gewebezellensammler mit vorgeschalteter Filtereinrichtung;
- Figur 3: eine schematische Schnittdarstellung durch einen Gehäuseabschnitt im Bereich der Fangeinrichtung;
- Figur 4: eine vergrößerte Darstellung eines plattenförmigen Körpers der Fangeinrichtung;
- Figur 5: eine schematische Schnittdarstellung durch einen Gewebezellensammler mit vorgeschalteter Fangeinrichtung und
- Figur 6: eine schematische Schnittdarstellung durch einen Gehäuseabschnitt der Fangeinrichtung.

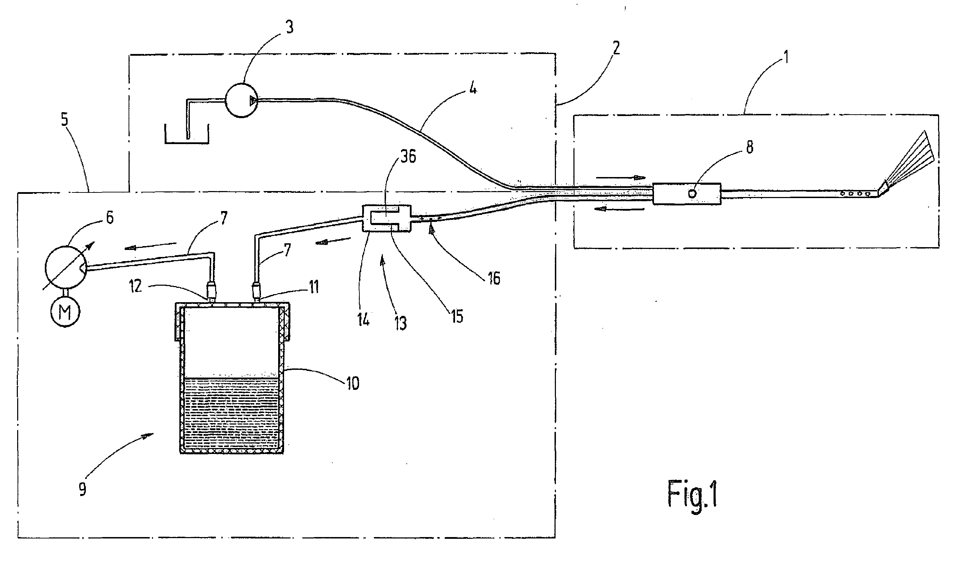

Figur 1 zeigt eine Einrichtung zum Flüssigkeitsstrahltrennen mit einem von der Hand eines Operateurs bedienbaren Applikator 1 zum wasserstrahlunterstützten Trennen und Absaugen von Gewebezellen aus einer biologischen Struktur. Die Einrichtung umfasst eine Druckstrahleinrichtung 2, mit einem Druckerzeuger 3 und einer Druckleitung 4 zur Versorgung des Applikators 1 mit einem definierten Flüssigkeitsstrahl. Die Einrichtung umfasst ferner eine Saugeinrichtung 5 zum Auffangen der abgetrennten Gewebeteile (Gewebezellen) und der verwendeten Arbeitsflüssigkeit und der körpereigenen Flüssigkeit aus der biologischen Struktur. Die Saugeinrichtung 5 umfasst einen Vakuumerzeuger 6 und eine Saugleitung 7, wobei die Saugleitung den Vakuumerzeuger 6 mit dem Applikator 1 durchgehend verbindet. Im Bereich des Applikators 1 besitzt die Saugleitung 7 einen verschließbaren Bypass 8, der die Saugleitung 7 mit der Atmosphäre verbindet. In der Saugleitung 7 befindet sich ein Restflüssigkeitssammler 9 für die abgesaugte Flüssigkeit mit einem verschließbaren Auffangbehälter 10 sowie einem Eingangsanschluss 11 und einem Ausgangsanschluss 12 für die Saugleitung 7. Der Restflüssigkeitssammler 9 für die ausgefilterte Flüssigkeit ist in Saugrichtung vor dem Vakuumerzeuger 6 angeordnet. Zwischen dem Restflüssigkeitssammler 9 und dem Applikator 1 ist ein Gewebezellensammler 13 in die Saugleitung 7 geschaltet. Der Gewebezellensammler 13 besteht aus einem Sammelbehälter 14, innerhalb dem ein Sieb 15 angeordnet ist, welches einen Sammelraum 36 abgrenzt. Dem Gewebezellensammler 13 ist eine Fangeinrichtung 16 vorgeschaltet.

Die in Figur 1 dargestellte Einrichtung zum Flüssigkeitsstrahltrennen zeigt folgende Funktion:

Bei Anwendung des Flüssigkeitsstrahltrennverfahrens tritt aus dem Applikator 1 ein definierter Flüssigkeitstrennstrahl aus, dessen Wirkung durch den in der Druckstrahleinrichtung erzeugten Flüssigkeitsdruck und die konstruktive Ausführung des Applikators 1 bestimmt wird. Diese Wirkung ist darauf gerichtet, Gewebezellen aus einer biologischen Struktur in schonender Weise herauszutrennen. Die so abgetrennten Gewebezellen werden zusammen mit der eingespritzten Arbeitsflüssigkeit und weiteren körpereigenen Flüssigkeiten durch ein in der Saugeinrichtung 5 erzeugtes Vakuum abgesaugt. Dieses Verfahren wird häufig bei der Fettabsaugung angewendet. Immer dann, wenn die derart abgesaugten Gewebezellen einer Wiederverwendung zugeführt werden sollen, werden diese Gewebezellen aus dem Flüssigkeits-Gewebezellen-Gemisch separiert. Dies geschieht durch den Gewebezellensammler 13.

Bei einer Stand-by-Stellung hält der Operateur den verschließbaren Bypass 8 geöffnet, so dass keine Absaugung erfolgt, sondern nur atmosphärische Luft angesaugt und durch den Gewebezellensammler 13 transportiert wird. Hierbei passiert die Luft im Gewebezellensammler 13 durch die Fangeinrichtung 16 den Sammelraum 36 und das Sieb 15 in Richtung Vakuumerzeuger 6.

In einer Betriebsstellung ist der verschließbare Bypass 8 durch den Operateur verschlossen, so dass sich die Saugkraft des Vakuumerzeugers 6 auf das Operationsfeld überträgt. Hierdurch werden die abgetrennten Gewebeteile (Gewebezellen) und die verschiedenen Flüssigkeiten aufgenommen und zum Gewebezellensammler 13 transportiert. Im Gewebezellensammler 13 gelangt dieses Gemisch aus Gewebezellen und Flüssigkeit in den Sammelraum 36 mit nachgelagertem Sieb 15. Die Gewebezellen werden mit dem Sieb 15 herausgefiltert, während die Flüssigkeit das Sieb 15 passiert und zum Restflüssigkeitssammler 9 gelangt.

Die Fangeinrichtung 16 sorgt dafür, dass in dem Flüssigkeits-Gewebezellen-Gemisch sich befindliche unerwünschte Gewebeteile, insbesondere Bindegewebe oder dergleichen, aufgefangen werden und nicht mit in den Sammelraum 36 zum Sieb 15 des Gewebesammelbehälters 13 gelangen. Die im Gewebesammelbehälter 13 somit gesammelten Gewebezellen (insbesondere Fettzellen) sind somit im Wesentlichen frei von unerwünschten Gewebeanteilen und von Flüssigkeit. Die gesammelten Gewebezellen können dann dem Gewebezellensammler 13/Sammelraum 36 entnommen und einer weiteren Verarbeitung zugeführt werden. Diese kann beispielsweise in einer Reinjektion in die biologische Struktur, aus der die Gewebezellen entnommen wurden, bestehen.

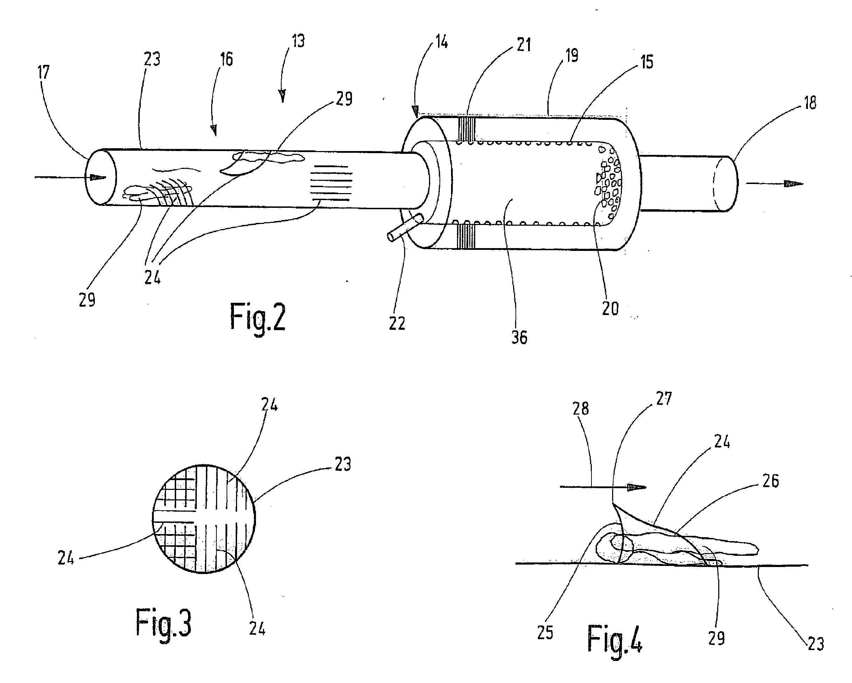

Figur 2 zeigt in einer schematischen Schnittdarstellung den Gewebezellensammler 13. Der Gewebezellensammler 13 besitzt einen Eingang 17, über den der Gewebezellensammler 13 mit dem Applikator 1 verbunden ist. Ferner ist ein Ausgang 18 vorgesehen, über den der Gewebezellensammler 13 mit dem Restflüssigkeitssammler 9 verbunden ist. Die Einbindung des Gewebezellensammlers 13 in die Saugleitung 7 kann über im Einzelnen nicht dargestellte Schnellverschlüsse, Steckverbinder oder dergleichen erfolgen. Wichtig ist, dass der Strömungsweg des zwischen Applikator 1 abgesaugten Flüssigkeits-Gewebezellen-Gemisches in Richtung des Gewebezellensammlers 13 und dann weiter in Richtung des Restflüssigkeitssammlers 9 gewährleistet ist. Der Sammelbehälter 14 besitzt eine Aufweitung 19, innerhalb der das Sieb 15 angeordnet. Das Sieb 15 wird beispielsweise durch ein Feinsieb gebildet. Das Feinsieb kann aus Kunststoff, Edelstahl oder dergleichen bestehen. Die Maschenweite des Siebes 15 ist so eingestellt, dass durch das Sieb 15 aus dem eintretenden Gewebezellen-Flüssigkeits-Gemisch die Gewebezellen 20, insbesondere Fettzellen, zurückgehalten werden, während die Flüssigkeit über den Ausgang 18 weiter zum Restflüssigkeitssammler 9 gelangt. Das Sieb 15 ist über eine Klemmeinrichtung/Fixierung 21 oder dergleichen in der Aufweitung/Ringspalt 19 des Gehäuses des Sammelbehälters 14 gehalten und bildet die Abgrenzung für den Sammelraum 36. Die Klemmeinrichtung/Fixierung 21 kann die Aufweitung/Ringspalt 19 komplett oder partiell verschließen. Durch den partiellen Verschluss ist ein Überströmen des Flüssigkeitszellen-Gemisches bei vollem Sammelraum 36 in die Aufweitung/Ringspalt 19 und weiter zum Ausgang 18 möglich, ohne das Sieb 15 passieren zu müssen. Durch die Auswahl/Dimensionierung der Querschnittsgrößen vom partiellen Verschluss der Aufweitung/Ringspalt 19 in Bezug zur Größe des Siebes 15 lässt sich optimalerweise eine Hauptströmungsrichtung einstellen. Die Hauptströmungsrichtung geht vorzugsweise durch den Sammelraum 36 und das Sieb 15. Erst bei vollem Sammelraum 36 wechselt die Hauptströmungsrichtung über die partiell verschlossene Aufweitung/Ringspalt 19 zum Ausgang 18. Der Sammelbehälter 14 besitzt ferner eine hier angedeutete verschließbare Entnahmeöffnung 22, über die mittels einer geeigneten Einrichtung die innerhalb des Sammelraumes 36 gesammelten Gewebezellen 20 abgezogen werden können.

Der Gewebezellensammler 13 umfasst ferner die Fangeinrichtung 16, die innerhalb eines Gehäuseabschnittes 23 angeordnet ist. Die Fangeinrichtung 16 besteht aus plattenförmigen Körpern 24, die in Gruppen - hier drei Gruppen - an der Innenwandung des Gehäuseabschnittes 23 angeordnet sind. Die plattenförmigen Körper 24 erstrecken sich jeweils radial nach innen in den Gehäuseabschnitt 23. Diese radiale Erstreckung reicht in etwa bis zu einer gedachten Mittellinie des Gehäuseabschnittes 23. Jede Gruppe der Körper der plattenförmigen Körper 24 besitzt mehrere, parallel zueinander angeordnete plattenförmige Körper 24, deren Ebenen in Strömungsrichtung des Flüssigkeits-Gewebezellen-Gemisches verlaufen. Hierdurch bilden die plattenförmigen Körper 24 eine so genannte Bartenstruktur, über die das in den Gewebezellensammler 13 eintretende Flüssigkeits-Gewebezellen-Gemisch gefiltert wird. Die drei Gruppen plattenförmiger Körper 24 sind hierbei in Strömungsrichtung hintereinander angeordnet. Ferner erstrecken sich die plattenförmigen Körper 24 jeweils koaxial versetzt von der Innenwandung des Gehäuseabschnittes 23 in Richtung der gedachten Mittellinie. Gemäß der Darstellung in Figur 2 erstreckt sich erst eine erste Gruppe der plattenförmigen Körper 24 von unten, anschließend eine nächste Gruppe der plattenförmigen Körper 24 von oben (um 180° versetzt) und schließlich eine dritte Gruppe der plattenförmigen Körper 24 seitlich (um 90° versetzt) jeweils in den Innenraum des Gehäuseabschnittes 23.

Nicht dargestellt im Ausführungsbeispiel können auch lediglich zwei Gruppen der plattenförmigen Körper 24 oder auch mehr als drei Gruppen der plattenförmigen Körper 24 vorgesehen sein. Denkbar ist auch, dass die plattenförmigen Körper 24 auf gleicher Höhe angeordnet sind und kammartig ineinandergreifen.

In Figur 3 ist schematisch eine Schnittdarstellung durch den Gehäuseabschnitt 23 gezeigt, wobei hier aus Richtung des Einganges 17 in den Gehäuseabschnitt 23 geblickt wird. Es wird die Anordnung der plattenförmigen Körper 24 deutlich. Hierbei ist die erste Gruppe der plattenförmigen Körper 24 unten, die zweite Gruppe der plattenförmigen Körper 24 oben und die dritte Gruppe der plattenförmigen Körper 24 links (jeweils entsprechend der Darstellung in Figur 3) angeordnet. Es ergibt sich somit eine überlagerte Gitterstruktur, wobei ein Strömungsquerschnitt innerhalb des Gehäuseabschnittes 23 durch die plattenförmigen Körper 24 der Filtereinrichtung 16 nur unwesentlich beeinträchtigt wird.

Figur 4 zeigt eine schematische vergrößerte Darstellung eines sich von der Innenwandung des Gehäuseabschnittes 23 erstreckenden plattenförmigen Körpers 24. Anhand der Darstellung in Figur 4 wird deutlich, dass der einzelne plattenförmige Körper 24 sich radial nach innen verjüngt und eine erste Flanke 25 und eine zweite Flanke 26 zu einer hakenförmigen Spitze 27 zusammengeführt sind. Die erste Flanke 25 ist entgegen der Strömungsrichtung 28 konkav ausgebildet, während die zweite Flanke 26 in der Strömungsrichtung 28 konvex ausgebildet ist. Die Darstellung ist lediglich beispielhaft. Auch andere Formgebungen der Fangstrukturen sind möglich, so zum Beispiel von der Hauptachse der Fangeinrichtung 16 in Strahlen vom Zentrum nach außen zur Wandung gerichtet.

Anhand der Darstellung in den Figuren 2, 3 und 4 wird deutlich, dass mittels der Fangeinrichtung 16 aus dem Flüssigkeits-Gewebezellen-Gemisch vor Eintritt in den Sammelraum 36 unerwünschte Gewebeteile, insbesondere Bindegewebe oder dergleichen, aufgefangen werden können. In den Figuren 2 und 4 sind hier relativ großformatige Bindegewebeteile 29 angedeutet, die sich in den bartenartigen Strukturen der plattenförmigen Körper 24 verfangen/hängenbleiben. Den Sammelraum 36 mit Sieb 15 erreicht dann nunmehr nur noch das zuvor gereinigte Flüssigkeits-Gewebezellen-Gemisch, so dass die vom Sieb 15 herausgefilterten Gewebezellen 20 keine unerwünschten Gewebeanteile enthalten.

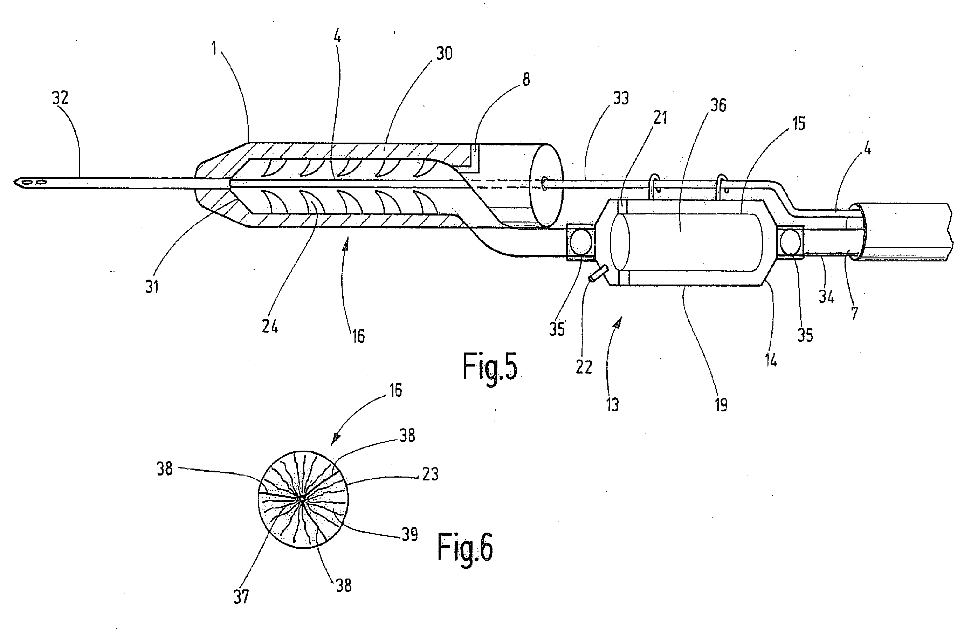

Figur 5 zeigt eine Ausführungsvariante der Erfindung, wobei gleiche Teile wie in den vorhergehenden Figuren - trotz teilweise unterschiedlicher Anordnung - mit gleichen Bezugszeichen versehen sind.

Bei der in Figur 5 gezeigten Ausführungsvariante ist die Fangeinrichtung 16 in einen Handgriff 30 des Applikators 1 integriert. Der Handgriff 30 besitzt hierzu einen entsprechend ausgebildeten Kanal 31, innerhalb dem die plattenförmigen Körper 24 angeordnet sind. Der Kanal 31 ist mit einem eigentlichen Operationsinstrument 32 verbunden. Dieses dient der Zuführung der Arbeitsflüssigkeit (hier nicht dargestellt) und der Absaugung des Flüssigkeits-Gewebezellen-Gemisches in an sich bekannter Weise.

Die Saugleitung 7 ist über einen Abschnitt 34 mit dem Sammelbehälter 14 verbunden, innerhalb dem - wie bereits erläutert - das Sieb 15 angeordnet ist.

Mit dem Bezugzeichen 33 ist eine Druckleitung zum Zuführen der Arbeitsflüssigkeit angedeutet.

Der Gewebezellensammler 13 ist über hier angedeutete Schnellverschlüsse 35 lösbar in den Abschnitt 34 eingebunden. Dies bedeutet, der Gewebezellensammler 13 ist austauschbar angeordnet. Hierdurch wird möglich, den Applikator 1 mit gegebenenfalls unterschiedlich aufgebauten Gewebezellensammlern 13 zu kombinieren. Auch kann vorgesehen sein, anstelle des Gewebezellensammlers 13 ein durchgehendes Saug- beziehungsweise Leitungsstück einzusetzen, so dass der Applikator 1 auch einsetzbar ist, wenn kein Gewebe gesammelt werden soll. Der Applikator 1 selber ist im Regelfall ein Einwegteil, das heißt, dieser wird nach einer bestimmungsgemäßen Verwendung entsorgt. Insofern ist das Vorhandensein der Fangeinrichtung 16 auch in dem Falle, dass kein Gewebe gesammelt werden soll, kein zusätzlicher Nachteil.

Die in Figur 5 dargestellte Einrichtung zeigt folgende Funktion:
In Stand-by-Stellung ist der verschließbare Bypass 8 geöffnet. Somit liegt der Unterdruck des Vakuumerzeugers 6 über den Abschnitt 34 sowie den Gewebezellensammler 13, den Schnellverschluss 35 und Bypass 8 an Atmosphäre an. Wird der Bypass 8 durch den Operateur verschlossen, liegt der Unterdruck über den Abschnitt 34 sowie den Gewebezellensammler 13 an dem Operationsinstrument 32 an. Das Flüssigkeits-Gewebezellen-Gemisch, das über das Operationsinstrument 32 aufgesaugt wird, gelangt dann über die Fangeinrichtung 16, den Sammelbehälter 14 in den Sammelraum 36. Durch das Sieb 15 gelangt dann nur die Restflüssigkeit in den Restflüssigkeitssammler 9 (Figur 1).

Figur 6 verdeutlicht schematisch eine Schnittdarstellung durch einen Gehäuseabschnitt 23 der Fangeinrichtung 16. Bei dieser Ausführungsform ist ein zentraler Haltekörper 37 vorgesehen, der sich koaxial zum Gehäuseabschnitt 23 erstreckt. Der Haltekörper 37 ist beispielsweise über hier angedeutete Stege 38 mit dem Gehäuseabschnitt 23 verbunden. Vom Haltekörper 37 erstrecken sich Fangstrukturen in Richtung der Wandung des Gehäuseabschnittes 23. Die Fangstrukturen werden von strangförmigen Körpern gebildet, die bürstenartig innerhalb des Gehäuseabschnittes 23 angeordnet sind. Diese Strukturen werden dann von dem Flüssigkeits-Gewebezellen-Gemisch durchströmt, so dass die Gewebezellen und die Flüssigkeit passieren können, während die Bindegewebeteile 29 sich in den Fangstrukturen verfangen und somit nicht in den Sammelraum 36 beziehungsweise zum Sieb 15 gelangen können. Die Anordnung der strangförmigen Körper 39 kann beabstandet in mehreren Reihen oder spiralförmig an dem Haltekörper 37 erfolgen.

### Bezugszeichenliste

- 1: Applikator
- 2: Druckstrahleinrichtung
- 3: Druckerzeuger
- 4: Druckleitung
- 5: Saugeinrichtung
- 6: Vakuumerzeuger
- 7: Saugleitung
- 8: Bypass
- 9: Restflüssigkeitssammler
- 10: Auffangbehälter
- 11: Eingangsanschluss
- 12: Ausgangsanschluss
- 13: Gewebezellensammler
- 14: Sammelbehälter
- 15: Sieb
- 16: Fangeinrichtung,
- 17: Eingang
- 18: Ausgang
- 19: Aufweitung/Ringspalt
- 20: Gewebezellen
- 21: Klemmeinrichtung/Fixierung
- 22: Entnahmeöffnung
- 23: Gehäuseabschnitt
- 24: plattenförmige Körper
- 25: erste Flanke
- 26: zweite Flanke
- 27: hakenförmige Spitze
- 28: Strömungsrichtung
- 29: Bindegewebeteile
- 30: Handgriff
- 31: Kanal
- 32: Operationsinstrument / Saugrohr mit koaxialer Druckleitung
- 33: Abschnitt / Druckschlauch
- 34: Abschnitt / Vakuumschlauch
- 35: Schnellverschlüsse
- 36: Sammelraum
- 37: zentraler Haltekörper
- 38: Stege
- 39: strangförmige Körper

## Patentansprüche

1. Vorrichtung zum flüssigkeitsstrahlunterstützten Trennen und Absaugen von Gewebezellen aus einer biologischen Struktur, mit einem Druckerzeuger zum Versorgen eines Applikators der Vorrichtung mit einem definierten Flüssigkeitsstrahl, mit einer Saugeinrichtung zum Entfernen der abgetrennten Gewebezellen und der verwendeten Flüssigkeit aus der biologischen Struktur, wobei die Saugeinrichtung eine Unterdruckquelle und eine Saugleitung zum Verbinden des Applikators mit der Unterdruckquelle umfasst, wobei in die Saugleitung ein Gewebezellensammler eingebunden ist, **dadurch gekennzeichnet, dass** dem Gewebezellensammler (13) in der Saugleitung (7) eine Fangeinrichtung (16) zum Auffangen von Bindegewebe oder dergleichen aus dem Flüssigkeits-Gewebezellen-Gemisch vorgeschaltet ist, wobei die Fangeinrichtung in einen Handgriff (30) des Applikators (1) integriert ist.

2. Vorrichtung nach Anspruch 1, **dadurch gekennzeichnet, dass** die Fangeinrichtung (16) in den Strömungsweg des Flüssigkeits-Gewebezellen-Gemisches einkragende Fangstrukturen aufweist.

3. Vorrichtung nach Anspruch 2, **dadurch gekennzeichnet, dass** die Fangstrukturen von Bartenstrukturen gebildet sind.

4. Vorrichtung nach einem der vorhergehenden Ansprüche, **dadurch gekennzeichnet, dass** die Bartenstrukturen von plattenförmigen Körpern (24) gebildet sind, deren Ebenen in Strömungsrichtung des Flüssigkeits-Gewebezellen-Gemisches orientiert sind.

5. Vorrichtung nach einem der vorhergehenden Ansprüche, **dadurch gekennzeichnet, dass** die plattenförmigen Körper (24) sich radial nach innen verjüngen.

6. Vorrichtung nach einem der vorhergehenden Ansprüche, **dadurch gekennzeichnet, dass** die plattenförmigen Körper (24) sich bis zu einer gedachten Mittellinie der Saugleitung erstrecken.

7. Vorrichtung nach einem der vorhergehenden Ansprüche, **dadurch gekennzeichnet, dass** in Strömungsrichtung hintereinander wenigstens zwei Gruppen von plattenförmigen Körpern (24) angeordnet sind.

8. Vorrichtung nach einem der vorhergehenden Ansprüche, **dadurch gekennzeichnet, dass** die Gruppen der plattenförmigen Körper (24) koaxial zueinander versetzt angeordnet sind.

9. Vorrichtung nach einem der vorhergehenden Ansprüche, **dadurch gekennzeichnet, dass** die Fangstrukturen von der Hauptachse der Fangeinrichtung (16) in Strahlen vom Zentrum nach außen zur Wandung gerichtet, in nachfolgenden Reihen angeordnet vorzugsweise spiralförmig gedreht sind.

10. Vorrichtung nach Anspruch 9, **dadurch gekennzeichnet, dass** die Fangstrukturen von Bürsten gebildet sind.

## Claims

1. An apparatus for fluid-jet assisted separation and aspiration of tissue cells from a biological structure, with a pressure generator for supplying a defined fluid jet to an applicator of the apparatus, with a suction device for removing the separated tissue cells and the used fluid from the biological structure, wherein the suction device comprises a vacuum source and a suction line for connecting the applicator to the vacuum source, wherein a tissue cell collector is incorporated in the suction line, **characterized in that** a catching device (16) for catching connective tissue or the like from the fluid/tissue cell mixture is arranged upstream of the tissue cell collector (13) in the suction line (7), wherein the catching device is integrated into a handle (30) of the applicator (1).

2. The apparatus according to claim 1, **characterized in that** the catching device (16) has catching structures protruding into the flow path of the fluid/tissue-cell mixture.

3. The apparatus according to claim 2, **characterized in that** the catching structures are formed by baleen structures.

4. The apparatus according to any one of the preceding claims, **characterized in that** the baleen structures are formed by plate-shaped bodies (24), the planes of which are oriented in the flow direction of the fluid/tissue-cell mixture.

5. The apparatus according to any one of the preceding claims, **characterized in that** the plate-shaped bodies (24) taper radially inwardly.

6. The apparatus according to any one of the preceding claims, **characterized in that** the plate-shaped bodies (24) extend up to an imaginary center line of the suction line.

7. The device according to any one of the preceding claims, **characterized in that** at least two groups of plate-shaped bodies (24) are arranged consecutively in the direction of flow.

8. The apparatus according to any one of the preceding claims, **characterized in that** the groups of the plate-shaped bodies (24) are arranged to be coaxially offset with respect to one another.

9. The apparatus according to any one of the preceding claims, **characterized in that** the catching structures are oriented in rays from the main axis of the catching device (16) from the center outwardly to the wall, in sequential rows preferably twisted in a spiral shape.

10. The apparatus according to claim 9, **characterized in that** the catching structures are formed by brushes.

## Revendications

1. Dispositif de séparation par jet de liquide et d'aspiration de cellules tissulaires à partir d'une structure biologique, avec un générateur de pression destiné à alimenter un applicateur du dispositif avec un jet de liquide défini, avec un dispositif d'aspiration pour l'enlèvement des cellules tissulaires séparées et du liquide utilisé hors de la structure biologique, le dispositif d'aspiration comprenant une source de dépression et une conduite d'aspiration pour le raccordement de l'applicateur à la source de dépression, un collecteur de cellules tissulaires étant inséré dans la conduite d'aspiration, **caractérisé en ce que**, en amont du collecteur de cellules tissulaires (13), un dispositif de retenue (16) est installé pour retenir du tissu conjonctif ou similaire à partir du mélange liquide-cellules tissulaires, le dispositif de retenue étant intégré dans une poignée (30) de l'applicateur (1).

2. Dispositif selon la revendication 1, **caractérisé en ce que** le dispositif de retenue (16) présente des structures de retenue faisant saillie dans le chemin d'écoulement du mélange liquide-cellules tissulaires.

3. Dispositif selon la revendication 2, **caractérisé en ce que** les structures de retenue sont formées de structures en fanons.

4. Dispositif selon une des revendications précédentes, **caractérisé en ce que** les structures en fanons sont formées de corps (24) en forme de plaque dont les plans sont orientés dans la direction d'écoulement du mélange liquide-cellules tissulaires.

5. Dispositif selon une des revendications précédentes, **caractérisé en ce que** les corps (24) en forme de plaque se rétrécissent radialement vers l'intérieur.

6. Dispositif selon une des revendications précédentes, **caractérisé en ce que** les corps (24) en forme de plaque s'étendent jusqu'à une ligne médiane imaginaire de la conduite d'aspiration.

7. Dispositif selon une des revendications précédentes, **caractérisé en ce qu'**au moins deux groupes de corps (24) en forme de plaque sont disposés l'un derrière l'autre dans la direction d'écoulement.

8. Dispositif selon une des revendications précédentes, **caractérisé en ce que** les groupes de corps (24) en forme de plaque sont disposés de façon décalée coaxialement les uns par rapport aux autres.

9. Dispositif selon une des revendications précédentes, **caractérisé en ce que** les structures de retenue de l'axe principal du dispositif de retenue (16) dirigées en jets à partir du centre vers l'extérieur en direction de la paroi sont disposées en rangées successives, tournées de préférence en forme de spirale.

10. Dispositif selon la revendication 9, **caractérisé en ce que** les structures de retenue sont formées de brosses.
